# EUROPÄISCHE PATENTSCHRIFT

(11) **EP 0 633 473 B1**
(45) Veröffentlichungstag und Bekanntmachung des Hinweises auf die Patenterteilung: **10.03.1999**
(21) Anmeldenummer: 93110471.5
(22) Anmeldetag: 30.06.1993
(51) Int. Cl.: G01N 33/86, C12Q 1/56

(54) **Messung des aktivierten partiellen Thromboplastinzeit (APTT) in einer Einstufen-Reaktion**
Measurement of the activated partial thromboplastin time (APTT) in a single-step reaction
La mesure du temps de thromboplastine partielle activée (TTPA) dans une réaction à étape-unique

(43) Veröffentlichungstag der Anmeldung: 11.01.1995
(73) Patentinhaber: STIFTUNG FÜR DIAGNOSTISCHE FORSCHUNG, CH-1785 Cressier sur Morat (CH)
(72) Erfinder: Josef, Dieter, CH-1785 Cressier (CH)
(74) Vertreter: Huber, Bernhard, Dipl.-Chem.

(56) Entgegenhaltungen:
- EP-A- 0 123 883
- EP-A- 0 394 070
- EP-A- 0 406 971
- EP-A- 0 535 799
- WO-A-90/11368
- CLINICAL CHEMISTRY Bd. 37, Nr. 4 , 1991 Seiten 520 - 526 B.J.OBERHARDT ET AL. 'Dry Reagent technology for Rapid, Convenient Measurements of Blood Coagulation and Fibrinolysis.'
- CLINICAL CHEMISTRY Bd. 37, Nr. 7 , 1991 Seiten 1235 - 1244 G.A.E.PONJEE ET AL. 'One.Step Chromogenic Equivalent of Activated Partial Thromboplastin Time Evaluated for Clinical Application.'
- HAEMOSTASIS Bd. 19, Nr. 1 , 1989 Seiten 13 - 20 J.HARENBERG ET AL. 'Comparative Study on a New One-Stage Clotting Assay for Heparin and Its Low Molecular Weight Derivatives.'

## Beschreibung

Die vorliegende Erfindung betrifft ein Verfahren und ein Reagenz zur Bestimmung der aktivierten partiellen Thromboplastinzeit (APTT) in einer Einstufen-Reaktion. Das erfindungsgemäße Verfahren und Reagenz eignet sich insbesondere für Bestimmungen, bei denen die APTT zusammen mit Bestimmungen weiterer Parameter des Blutgerinnungssystems durchgeführt wird.

Die Bestimmung der aktivierten partiellen Thromboplastinzeit (APTT) gehört zu den am häufigsten durchgeführten Messungen in der Gerinnungsanalytik. Hauptsächlich dient sie dazu, im Plasma von Patienten Faktorenmangel - wie Fehlen oder Verminderung des Faktors VIII - aufzudecken und zur Kontrolle der Heparintherapie.

Die Analyse wird üblicherweise wie folgt durchgeführt: eine Probe des Patientenplasmas wird mit einem Aktivator versetzt. Während einer Inkubationsphase wird der Faktor XII aktiviert. Anschließend wird die Reaktion mit Calciumchlorid gestartet. Gemessen wird die Zeitspanne bis eine Gerinnung der Probe eintritt.

Reagenzien zur Durchführung dieses Verfahrens sind schon längere Zeit bekannt und werden z.B. durch die Firmen Baxter, Ortho und DiaMed vertrieben. Als Aktivator werden üblicherweise Kaolin, Silicat oder Elagsäure verwendet.

Die eigentliche Messung erfolgt durch manuelles Kippen der Analysenröhrchen und visueller Beobachtung der Gerinnselbildung. Die Gerinnungszeit kann aber auch mit optischen oder mechanischen Meßgeräten bestimmt werden, wie sie z.B. von den Firmen Amelung, Baxter, Labor und Behring vertrieben werden.

Das oben beschriebene übliche Verfahren weist jedoch einige gravierende Nachteile auf:
1. Die Aktivierungsphase muß für alle Patienten als konstant angenommen werden, so daß patientenspezifische Besonderheiten bei der Messung nicht berücksichtigt werden können. In dieser Aktivierungsphase beginnt jedoch die für die Blutungs- oder Thromboseneigung der Patienten mitentscheidende Aktivierung des endogenen Gerinnungssystems und die Fibrinolyse. So führt eine zu kurze Inkubationszeit schon bei normalen Plasmen - weil die Aktivierung unvollständig ist - zu verlängerten Zeiten. Ebenfalls verlängern sich die Werte bei zu langer Inkubationszeit, weil das aktivierte fibrinolytische System aus dem Fibrinogen Spaltprodukte freisetzt, die die Gerinnung verzögern.
2. Ein zweiter Nachteil des herkömmlichen Verfahrens ist, daß die Analyse in zwei Stufen erfolgen muß, was die Fehlermöglichkeiten erhöht und die Standardisierung der Bestimmungsmethoden erschwert.
3. Weiterhin ist die insgesamt schlechte Standardisierbarkeit der jetzigen APTT-Bestimmung ein wesentlicher Nachteil der bestehenden Methode. Die Meßergebnisse werden üblicherweise in Sekunden erfaßt, bei der Herstellung des Reagenz treten aber zwangsläufig Schwankungen auf, da es sich bei den Aktivatoren nicht um exakt definierbare chemische Substanzen mit immer gleicher Oberlfäche handelt. Außerdem ist es schwierig, die für den Gerinnungsablauf notwendigen Phospholipide in chemisch reiner Form zu gewinnen und deren Aktivität konstant zu halten. Die Folge sind zwangsläufig Variationen in den Meßergebnissen von Charge zu Charge. Die variierenden Resulatate werden - falls sie nicht zu groß sind - jedoch als naturgegeben in Kauf genommen.
4. Ein vierter Nachteil ist die schlechte Automatisierbarkeit der klassischen APTT-Bestimmung. Bei anderen wichtigen Gerinnungstests, wie Thromobplastinzeit (PT) und Fibrinogen, handelt es sich um schnelle Reaktionsabläufe, die mit einem einzigen Testreagenz durchgeführt werden können, ein Zweistufentest mit relativ langer Vorinkubationszeit paßt z.B. bei paralleler Bestimmung von mehreren Parametern des Gerinnungssystems zeitlich nur schlecht in die Analysenabläufe und verringert stark den Analysenfluß.
5. Wünschenswert ist ferner das Starten der Reaktion mit Plasma, da dadurch Testausführung, Berechnung und Analyzer-Funktionen wesentlich vereinfacht werden könnten.

G. Ponjee et al. (Clin. Chem., 37 (7) (1991) 1235 bis 1244) beschreibt einen Vergleich der chromogenen Methode und der herkömmlichen zweistufigen Methode zur APTT-Bestimmung. Bei der chromogenen Methode wird der Probe ein chromogenes Substrat für Thrombin zugegeben, wobei die Farbentwicklung durch Spaltung des chromogenen Substrats bestimmt wird. Auch EP-A-0 123 883 betrifft ein Verfahren zur photometrischen Bestimmung der aktivierten partiellen Thromboplastinzeit, in welchem ein chromogenes Substrat verwendet wird.

EP 0 394 070 A1 beschreibt die sog. Vollblut-Methode ohne Antigoagulant in einem Kapillarsystem. Diese Methode wird häufig für die Kontrolle einer Therapie durch den Arzt verwendet, wobei dem Patienten mit einer Kapillare ein Tropfen Blut abgenommen wird, der sofort analysiert wird. Der Test muß innerhalb weniger Minuten nach der Blutabnahme erfolgen.

Oberhardt et al., Clinical Chemistry, Band 37, Nr. 4, 1991, Seiten 520 bis 526, beschreibt ein Verfahren unter Verwendung einer Reagenzienmischung in trockener Form. Das beschriebene Verfahren basiert auf der Bewegung von paramagnetischen Eisenoxidpartikeln in einem oszillierenden Magnetfeld, wobei die Abnahme der Oszillation gemessen wird. Die für dieses Verfahren benötigten Materialien und Testvorrichtungen sind sehr aufwendig und das Verfahren eignet sich nur bedingt für die Bestimmung einer größeren Anzahl von Proben.

Das der vorliegenden Erfindung zugrundeliegende technische Problem bestand somit darin, die Nachteile des Standes der Technik möglichst weitgehend zu vermeiden, d.h. insbesondere einen Einbezug der Aktivierungsphase in die APTT-Reaktion, eine Standardisierung der Ergebnisse, eine Verringerung der Zahl der Pipettierschritte und das Starten der Reaktion mit der Probeflüssigkeit, insbesondere Plasma, zu ermöglichen.

Dieses Problem wurde gelöst durch ein Verfahren zur Bestimmung der aktivierten partiellen Thromboplastinzeit (APTT) in einer Einstufen-Reaktion, welches dadurch gekennzeichnet ist, daß man die Bestimmung von APTT durch Kontaktieren eines Reagenz, das die zur APTT-Bestimmung erforderlichen Substanzen umfassend einen Aktivator, zweiwertige Kationen und Phosphatlipide in einem vorgemischten Zustand enthält, und welches durch eine oder mehrere Aminosäuren stabilisiert ist mit einer Probeflüssigkeit startet und daß man die APTT durch Messung der Gerinnungszeit bestimmt.

Bei dem erfindungsgemäßen Verfahren kann das Reagenz in einem Bestimmungsgefäß vorgelegt und dann die Reaktion durch Zugabe der Probeflüssigkeit gestartet werden. Andererseits ist es ebenso möglich, die Probeflüssigkeit vorzulegen und die Reaktion durch Zugabe des vorgemischten Reagenz zu starten. Wichtig ist, daß durch den direkten Kontakt von Reagenz und Probeflüssigkeit die Bestimmung gestartet wird, ohne daß die in den Verfahren des Standes der Technik erforderliche Aktivierungsphase benötigt wird.

Vorzugsweise wird die Bestimmung der APTT nach dem erfindungsgemäßen Verfahren durch Verwendung von gegebenenfalls verdünntem Blut oder Plasma, besonders bevorzugt durch Verwendung von Plasma als Probeflüssigkeit durchgeführt.

Das Reagenz zur Bestimmung der APTT enthält die zur Bestimmung von APTT erforderlichen Substanzen in einem vorgemischten Zustand. Bei den erforderlichen Substanzen handelt es sich im allgemeinen eine Aktivatorkomponente, zweiwertige Kationen und Phospholipide. Als Aktivator verwendet man vorzugsweise Kaolin, Silicat oder/und Elagsäure. Die Konzentrationen des Aktivators liegen z.B. bei vorzugsweise 25 bis 250 mg/l, besonders bevorzugt bei 100 bis 100 mg/l durch Glaswolle aktivierte Elagsäure pro Liter Reagenz. Die im Reagenz enthaltenen Phospholipide können aus tierischen oder/und pflanzlichen Gewebeextrakten gewonen oder/und synthetisch hergestellt werden. Ein Beispiel für geeignete Phospholipide sind z.B. Chloroformextrakte aus Kaninchenhirn. Pro Liter Reagenz wird der Extrakt vorzugsweise aus 0,2 bis 1,0 g, besonders bevorzugt aus 0,4 bis 0,6 g Kaninchenhirn verwendet. Bei den im Reagenz vorhandenen zweiwertigen Kationen handelt es sich vorzugsweise um Calciumionen, die beispielsweise durch Zusatz von Calciumchlorid zum Reagenz bereitgestellt werden. Die Konzentration der Calciumionen in der Bestimmungslösung beträgt vorzugsweise 5 bis 15 mmol/l, besonders bevorzugt 7,5 bis 12,5 mmol/l.

Weiterhin ist das Reagenz durch eine oder mehrere Aminosäuren stabilisiert. Durch diese Stabilisierung wird die Lagerbeständigkeit des Reagenz auch bei höheren Temperaturen (z.B. 37°C) ganz erheblich verbessert. Vorzugsweise werden zur Stabilisierung Aminosäuren verwendet, die aus der Gruppe, die D-Alanin, L-Alanin, β-Alanin, Glycin und Valin umfaßt, ausgewählt sind. Weiterhin ist bevorzugt, daß die Aminosäuren in einer Gesamtkonzentration von 0,5 bis 10% bezüglich des Reagenzgesamtgewichts vorliegen.

Überraschenderweise wurde festgestellt, daß entgegen allen bisherigen Testvorschriften eine einstufige Testdurchführung zur Bestimmung der APTT möglich ist. So können der Aktivator und die Phospholipide mit den zweiwertigen Kationen (z.B. Calciumchlorid) vermischt und diese Mischung mit Aminosäuren (z.B. Alanin) stabilisiert werden. Auf diese Weise wird ein vorgemischtes Reagenz erhalten, das alle zur Bestimmung der APTT erforderlichen Substanzen enthält. Das Starten der Reaktion ist nun durch direktes Kontaktieren der Probeflüssigkeit, z.B. Plasma, mit diesem Reagenz möglich. Überraschenderweise kann durch das erfindungsgemäße Verfahren die bisher als unbedingt erforderlich erachtete Aktivierungsphase weggelassen werden.

In einer bevorzugten Ausführungsform des erfindungsgemäßen Verfahrens liegt das Reagenz bereits fertig in Gefäßen, insbesondere Küvetten, abgepackt vor. Das Reagenz kann in fester oder flüssiger Form sein, z.B. als Lösung oder als Lyophilisat. Die Gefäße bestehen vorzugsweise aus einem dichten, transparenten Material, wie z.B. Polypropylen.

Weiterhin ist bevorzugt, daß die Gefäße nach der Füllung mit dem Reagenz luft- und wasserdicht verschlossen werden. Zu diesem Zweck kann man beispielsweise einen Verschluß verwenden, der aus einer Versiegelung mit Aluminiumfolie besteht. Auf diese Weise ist es möglich, die APTT-Bestimmung erheblich zu vereinfachen und in Analysensysteme zur Durchführung von Bestimmungen weiterer Parameter des Gerinnungssystems zu integrieren, indem die Gefäße mit den APTT-Reagenzien industriell abgepackt bzw. vorgefüllt werden.

Zu diesem Zweck ist es weiterhin bevorzugt, daß mehrere Gefäße in einer Karte, d.h. in einer gemeinsamen Halterung integriert sind. Auf dieser Halterung oder/und auf den Gefäßen selbst können reagenzien- oder/und chargenspezifische Karten gespeichert werden. Eine derartige Speicherung kann beispielsweise auf einem Strichcode-Etikett erfolgen.

Auf diese Weise können chargenspezifische Korrekturfaktoren des Reagenz eingesetzt werden, was die Standardisierung der Ergebnisse und damit deren Interpretierbarkeit erheblich erleichtert. Weiterhin kann das Pipettieren der Reagenzkomponenten im Labor vermieden werden, was eine Optimierung des Tests darstellt und ein stark vereinfachtes Computerprogramm zur Steuerung der Analysendurchführung und zur Auswertung der Ergebnisse ermöglicht, insbesondere bei einer industriellen Abpackung bzw. Vorfüllung der Meßgefäße, z.B. wenn die Meßgefäße aus einem dichten Material wie Polypropylen bestehen und mit einer Folie bzw. einem Deckel hermetisch verschlossen sind.

Die oben beschriebene Erfindung des Einstufentests zur APTT-Bestimmung ermöglicht ferner, auf einfachste Weise Patienten-profile zu erstellen, bei denen z.B. die Reagenzien zur Messung der Thromboplastinzeit, des Fibrinogens, der Thrombinzeit und der Reptilasezeit zusätzlich in einer Reagenzienkombination, z.B. einer Karte, bestehend aus mehreren Gefäßen, integriert sind. Die Adaption weiterer Gerinnungstests wie Gerinnungsfaktoren (z.B. Faktoren VIII, IX, X, XI, XII), Protein S und Protein C ist ebenfalls möglich. Zur Durchführung ist einzig das Starten der Reaktion mit dem Patientenplasma erforderlich. Alle testspezifischen Informationen wie Normalwerte, Eichkurven etc. können auf einem Strichcode gespeichert werden, dadurch ist die mit großen Fehlerquellen behaftete Bereitstellung eines Frisch-Normal-Plasmapools (FNP) und die direkte Erstellung der Eichkurven im Anwenderlabor nicht mehr notwendig. Bei einer automatischen Ausführung der Analyse erübrigt sich dazu die Reagenzienpipettierung und die aufwendige Logistik dafür. eine Kombination von codierten Reagenzien mit codierten Patientenproben eliminiert Verwechslungen und führt dadurch zu optimal zuverlässigen Resultaten.

Ein weiterer Gegenstand der vorliegenden Erfindung ist somit die Kombination des APTT-Bestimmungsverfahrens mit weiteren Bestimmungsmethoden, die parallel zur APTT-Bestimmung durchgeführt werden können. Beispiele hierfür sind weitere Parameter des Gerinnungssystems, beispielsweise die Thromboplastinzeit (PT), die Fibrinogenkonzentration, die Thrombinzeit (TT), die Reptilasezeit (RT), Gerinnungsfaktoren, Protein C und Protein S.

Die Erfindung betrifft auch ein Reagenz zur Bestimmung der aktivierten partiellen Thromboplastinzeit (APTT) in einer Einstufen-Reaktion, das alle zur Bestimung der APTT erforderlichen Substanzen, umfassend einen Aktivator, Phospholipide und zweiwertige Kationen, in vorgemischtem Zustand enthält, und durch eine oder mehrere Aminosäuren stabilisiert ist. Ein derartiges Reagenz enthält im allgemeinen, wie oben spezifiziert, Phospholipide, einen Aktivator und zweiwertige Kationen. Das Reagenz ist, wie oben spezifiziert, durch eine oder mehrere Aminosäuren stabilisiert. Das erfindungsgemäße Reagenz unterscheidet sich von den Reagenzien des Standes der Technik dadurch, daß es alle zur APTT-Bestimmung erforderlichen Substanzen in vorgemischtem Zustand enthält, während bei den bekannten Reagenzien mindestens eine zur Bestimmung von APTT erforderliche Komponente, meist die zweiwertigen Ionen, gefehlt hat.

Das Reagenz ist vorzugsweise in flüssiger oder fester Form (z.B. als Pulver oder Lyophilisat) in einem Gefäß abgepackt bzw. vorgefüllt und kann mehrere in einer Karte integrierte Gefäße umfassen, wobei reagenzien- oder/und chargenspezifische Daten auf den Gefäßen oder/und deren Halterung gespeichert sein können.

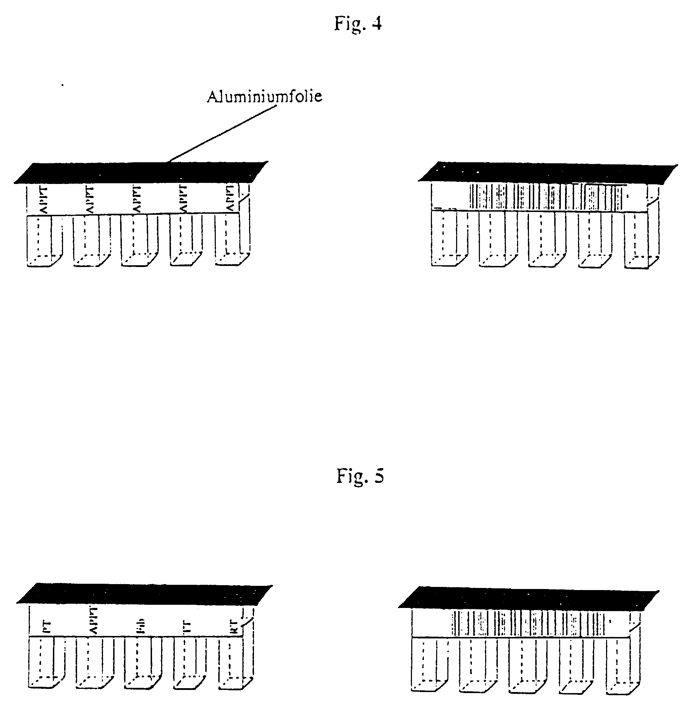

Hierzu kann man beispielsweise - wie in Figur 4 gezeigt - eine Karte mit fünf Meßküvetten für APTT-Tests verwenden, wobei auf der Karte oder/und auf den Meßküvetten selbst Daten, wie etwa der Verfall der Reagenzien, die Lotnummer, der Wert des Frisch-Normal-Plasmapools, die Umrechnung in andere Meßeinheiten und Korrekturfaktoren gespeichert werden können. Weiterhin ist es möglich, eine derartige Karte - wie in Figur 5 gezeigt - für ein Patientenprofil zu konzipieren, mit dem verschiedene Analysen (z.B. APTT, PT, Fibrinogen, TT und RT) parallel durchgeführt werden können.

Ein Gegenstand der vorliegenden Erfindung ist daher auch eine Reagenzienkombination, die mehrere Gefäße mit einem erfindungsgemäßen Reagenz zur APTT-Bestimmung und gegebenenfalls weitere Gefäße zur Bestimmung anderer Parameter des Gerinnungssystems umfaßt.

Anhand der Figuren werden gewisse Aspekte der Erfindung näher erläutert:
Es zeigen:
- Figur 1a: den zeitlichen Gerinnungsablauf bei der Bestimmungsmethode des Standes der Technik mit Normalplasma (Starten der Reaktion konventionell mit Calciumchlorid nach 3 Minuten Vorinkubation),
- Figur 1b: den zeitlichen Gerinnungsablauf bei der erfindungsgemäßen Bestimmungsmethode (Starten der Reaktion mit Normalplasma),
- Figur 2a: den zeitlichen Gerinnungsablauf bei der Bestimmungsmethode des Standes der Technik mit Faktor VIII-Mangelplasma (Starten der Reaktion mit Calciumchlorid nach 3 Minuten Vorinkubation),
- Figur 2b: den zeitlichen Gerinnungsablauf bei der erfindungsgemäßen Bestimmungsmethode (Starten der Reaktion mit Faktor VIII-Mangelplasma),
- Figur 3: die Korrelation beider Methoden mit verschiedenen Plasmen,
- Figur 4: die Vorder- und Rückseite einer Karte, in die fünf Meßküvetten integriert sind. Verfall der Reagenzien, Lotnummer, Wert des Frisch-Normal-Plasmapools, Umrechnung in andere Meßeinheiten und Korrekturfaktoren können auf dem Strichcode der Rückseite gespeichert werden,
- Figur 5: die Vorder- und Rückseite einer Karte, in die ebenfalls z.B. fünf Meßküvetten integriert sind. Diesmal ist die Karte für ein Patientenprofil konzipiert, mit dem verschiedene Analysen gleichzeitig durchgeführt werden können. Der Strichcode auf der Rückseite enthält alle Daten wie in Figur 4.

Die beschriebene Erfindung wird an folgenden Beispielen näher erläutert:

### BEISPIEL 1

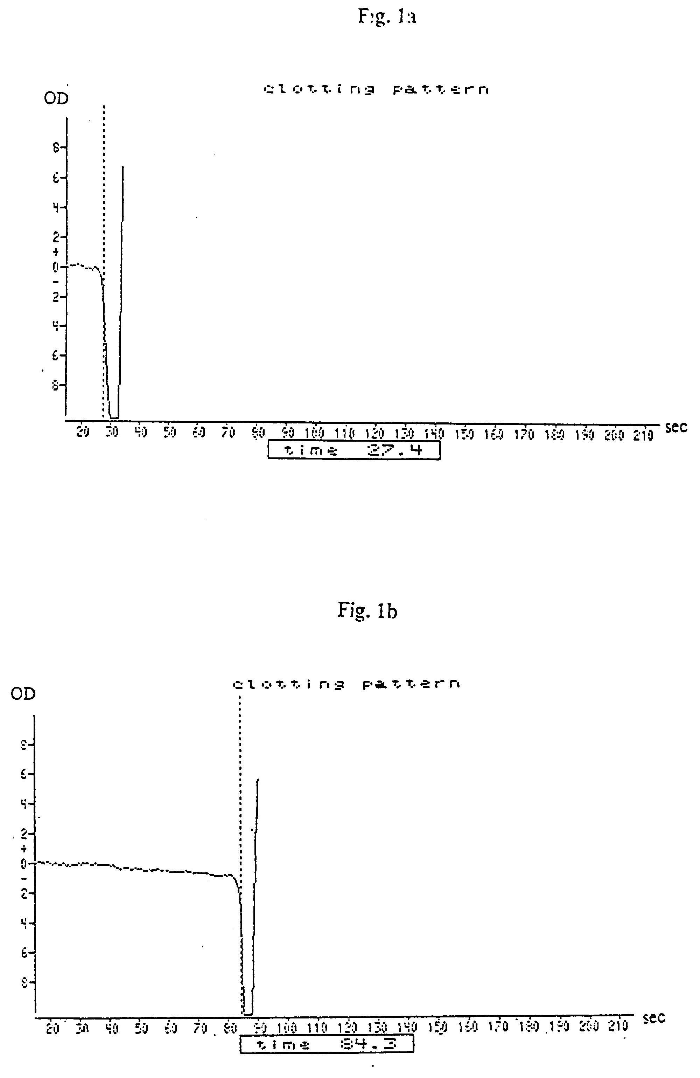

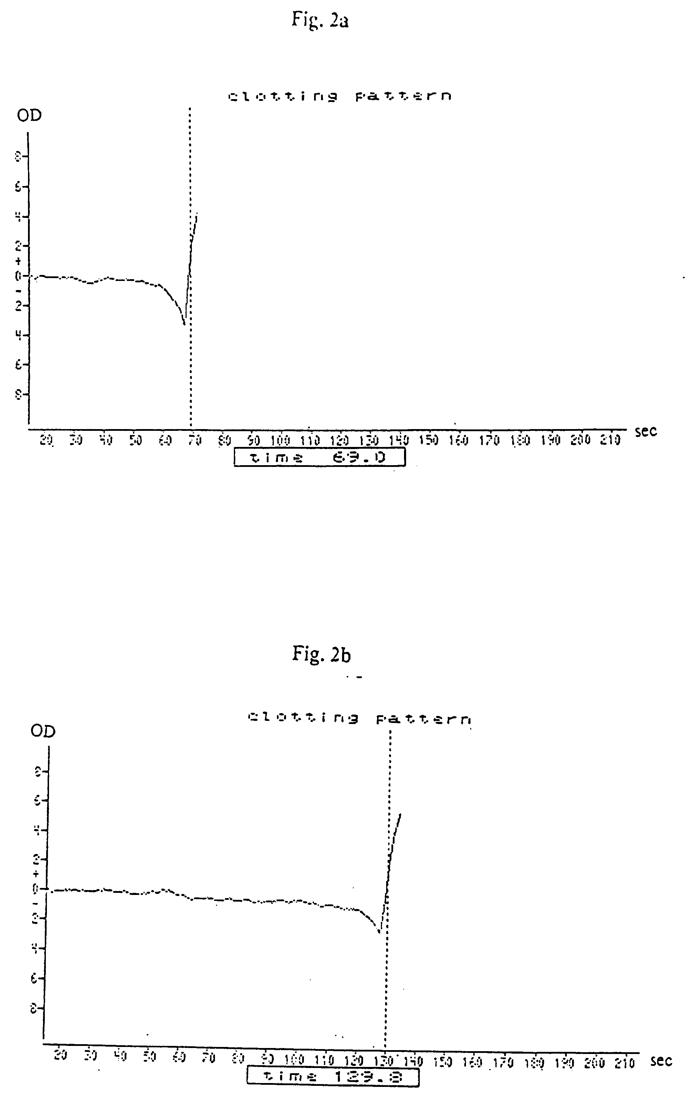

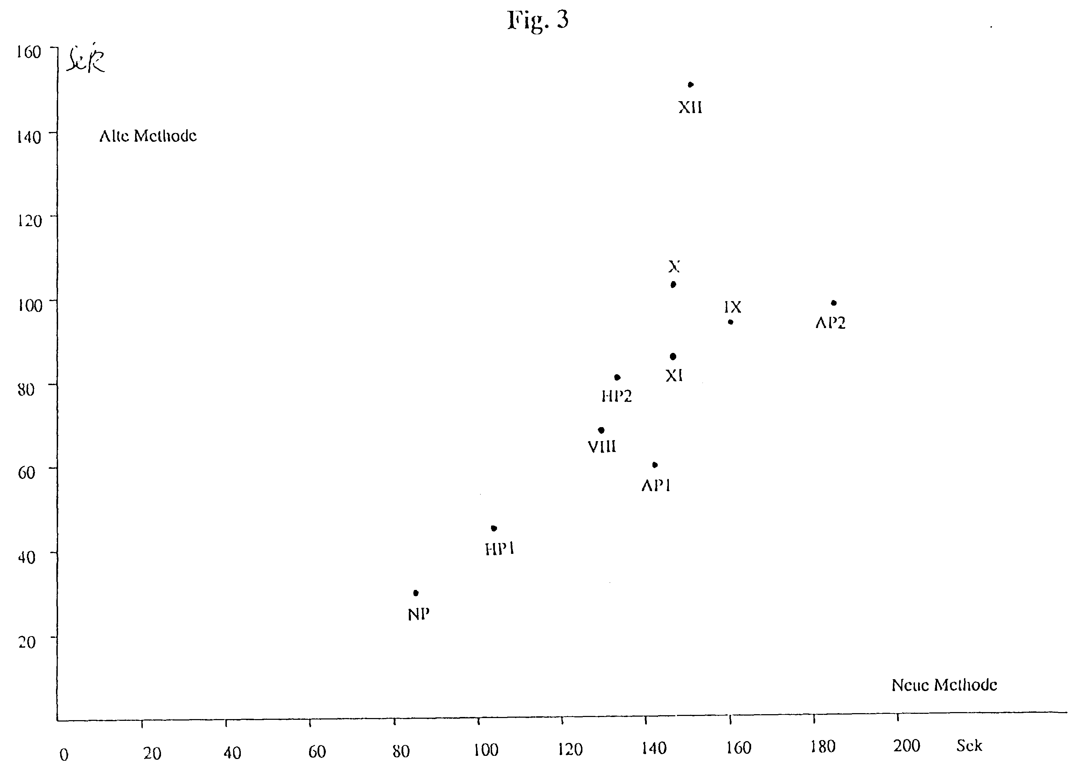

50 ml APTT-Reagenz (DiaCelin, DiaMed) werden mit 50 ml 0,02 mol/l Calciumchlorid vermischt. 0,2 ml dieser Mischung werden in Küvetten der Firma Labor pipettiert. Die Reaktion wird mit 0,1 ml Plamsa gestartet. Der Gerinnungsverlauf wird optisch in einem Analyzer der Firma Labor (Coascreener) verfolgt und registriert. Als Plasmen werden Normalplasma, Abnormalplasma (mittel und hoch), Heparinplasma (0,15 u/ml und 0,3 u/ml), Faktorenmangelplasmen (Faktor VIII, IX, X, XI, XII, Gehalt ca. 1 %) verwendet. Parallel dazu werden die Plasmen auf herkömmliche Weise mit den Reagenzien der Firma DiaMed entsprechend der Gebrauchsanweisung analysiert. Die Ergebnisse sind in Tabelle 1 zusammengefaßt, Figuren 1 und 2 zeigen zwei typische Gerinnungskurven (Normalplasma und Faktor VIII-Mangelplasma); Figur 3 die Korrelation beider Verfahren und Figur 4 eine geeignete Karte für mehrere Bestimmungen gleichzeitig.

**Tabelle 1**

| | Gerinnungszeit (sec) | |
|---|---|---|
| | Methode des Standes der Technik | erfindungsgemäße Methode |
| Normalplasma 1 (NP) | 27,5 | 85,4 |
| Abnormalplasma 1 (AP1) | 59,3 | 141,3 |
| Abnormalplasma 2 (AP2) | 97,7 | 184,2 |
| Heparinplasma 1 (HP1) | 46,3 | 104,2 |
| Heparinplasma 2 (HP2) | 80,5 | 132,5 |
| Faktormangelplasma VIII | 68,0 | 128,7 |
| Faktormangelplasma IX | 94,2 | 158,9 |
| Faktormangelplasma X | 103,5 | 145,0 |
| Faktormangelplasma XI | 86,1 | 145,4 |
| Faktormangelplasma XII | 150,9 | 151,9 |

### BEISPIEL 2

Das Experiment nach Beispiel 1 wird zur Verbesserung der Stabilität unter Zusatz der jeweils angegebenen Menge von Aminosäuren wiederholt. Der Stabilisierungsgrad bei verschiedenen Konzentrationen wird in einem Streß-Test durch Lagerung der Reagenz bei 37°C mit Normalplasma ermittelt. Die Ergebnisse sind in Tabelle 2 zusammengefaßt. D-Alanin, L-Alanin, β-Alanin und Glycin stabilisieren demnach das Reagenz sehr gut.

**Tabelle 2a**

| D-Alanin | Gerinnungszeit in sec. | | | |
|---|---|---|---|---|
| | Anfang | 3 Tage 37°C | 1 Woche 37°C | 2 Wochen 37°C |
| 0 % | 80 | 116 | 129 | 140 |
| 0,625 % | 75 | 96 | 102 | 117 |
| 1.25 % | 73 | 88 | 90 | 99 |
| 2.5 % | 72 | 81 | 82 | 85 |
| 5.0 % | 73 | 75 | 74 | 74 |

**Tabelle 2b**

| L-Alanin | Gerinnungszeit in sec. | | | |
|---|---|---|---|---|
| | Anfang | 3 Tage 37°C | 1 Woche 37°C | 2 Wochen 37°C |
| 0 % | 80 | 107 | 135 | 145 |
| 0,625 % | 75 | 91 | 103 | 119 |
| 1.25 % | 74 | 86 | 92 | 102 |
| 2.5 % | 75 | 80 | 83 | 86 |
| 5.0 % | 77 | 79 | 80 | 80 |

**Tabelle 2c**

| β-Alanin | Gerinnungszeit in sec. | | | |
|---|---|---|---|---|
| | Anfang | 3 Tage 37°C | 1 Woche 37°C | 2 Wochen 37°C |
| 0 % | 81 | 108 | 130 | 144 |
| 0,625 % | 76 | 96 | 107 | 118 |
| 1.25 % | 74 | 88 | 93 | 100 |
| 2.5 % | 75 | 74 | 82 | 83 |
| 5.0 % | 77 | 79 | 79 | 78 |

**Tabelle 2d**

| Glycin | Gerinnungszeit in sec. | | | |
|---|---|---|---|---|
| | Anfang | 3 Tage 37°C | 1 Woche 37°C | 2 Wochen 37°C |
| 0 % | 81 | 110 | 136 | 136 |
| 0,625 % | 76 | 92 | 98 | 103 |
| 1.25 % | 75 | 84 | 90 | 92 |
| 2.5 % | 75 | 79 | 82 | 80 |
| 5.0 % | 83 | 85 | 87 | 87 |

**Tabelle 2e**

| Valin | Gerinnungszeit in sec. | | | |
|---|---|---|---|---|
| | Anfang | 3 Tage 37°C | 1 Woche 37°C | 2 Wochen 37°C |
| 0 % | 86 | 111 | 130 | 145 |
| 0,625 % | 80 | 98 | 106 | 126 |
| 1.25 % | 79 | 91 | 95 | 105 |
| 2.5 % | 78 | 88 | 92 | 96 |
| 5.0 % | 82 | 91 | 94 | 95 |

**Tabelle 2f**

| Lysin | Gerinnungszeit in sec. | | | |
|---|---|---|---|---|
| | Anfang | 3 Tage 37°C | 1 Woche 37°C | 2 Wochen 37°C |
| 0 % | 78 | 108 | 131 | 140 |
| 0,625 % | 80 | 91 | 104 | 128 |
| 1.25 % | 93 | 106 | 120 | 118 |
| 2.5 % | 160 | 202 | --- | --- |
| 5.0 % | > 250 | --- | --- | --- |

**Tabelle 2g**

| Methionin | Gerinnungszeit in sec. | | | |
|---|---|---|---|---|
| | Anfang | 3 Tage 37°C | 1 Woche 37°C | 2 Wochen 37°C |
| 0 % | 81 | 108 | 125 | 140 |
| 0,625 % | 75 | 94 | 99 | 119 |
| 1.25 % | 76 | 90 | 95 | 110 |
| 2.5 % | 81 | 95 | 96 | 110 |
| 5.0 % | 105 | 122 | --- | --- |

**Tabelle 2h**

| Glutaminsäure | Gerinnungszeit in sec. | | | |
|---|---|---|---|---|
| | Anfang | 3 Tage 37°C | 1 Woche 37°C | 2 Wochen 37°C |
| 0 % | 82 | 110 | 131 | 142 |
| 0,625 % | 74 | 93 | 102 | 120 |
| 1.25 % | 72 | 86 | 91 | 102 |
| 2.5 % | 78 | 87 | 92 | 103 |
| 5.0 % | 122 | 160 | 168 | 180 |

**Tabelle 2i**

| N-Acetyl-Cystein | Gerinnungszeit in sec. | | | |
|---|---|---|---|---|
| | Anfang | 3 Tage 37°C | 1 Woche 37°C | 2 Wochen 37°C |
| 0 % | 83 | 117 | 137 | 142 |
| 0,625 % | 122 | 166 | 176 | >250 |
| 1.25 % | 228 | >250 | --- | --- |
| 2.5 % | >250 | --- | --- | --- |
| 5.0 % | >250 | --- | --- | --- |

**Tabelle 2j**

| Asparaginsäure | Gerinnungszeit in sec. | | | |
|---|---|---|---|---|
| | Anfang | 3 Tage 37°C | 1 Woche 37°C | 2 Wochen 37°C |
| 0 % | 81 | 109 | 135 | 136 |
| 0,625 % | 74 | 88 | 102 | 109 |
| 1.25 % | 73 | 83 | 90 | 92 |
| 2.5 % | 77 | 82 | 89 | 88 |
| 5.0 % | 110 | 118 | 140 | 130 |

**Tabelle 2k**

| Arginin | Gerinnungszeit in sec. | | | |
|---|---|---|---|---|
| | Anfang | 3 Tage 37°C | 1 Wochen 37°C | 2 Wochen 37°C |
| 0 % | 83 | 117 | 132 | 140 |
| 0,625 % | 83 | 85 | 125 | 141 |
| 1.25 % | 103 | 165 | 160 | 165 |
| 2.5 % | 214 | >250 | --- | --- |
| 5.0 % | >250 | --- | --- | --- |

### BEISPIEL 3

Das Experiment nach Beispiel 1 wird wiederholt, jedoch werden simultan die Thromboplastinzeit (PT), das Fibrinogen (Fib), die Thrombinzeit (TT) und die Reptilasezeit (RT) mitbestimmt. Verwendet werden die Reagenzien der Firma DiaMed. Die Reagenzien werden wie folgt verwendet:
a) DiaPlastin zur Messung der Thromoplastinzeit (PT)
   0,2 ml des Reagenz werden in die erste Küvette pipettiert. Die Reaktion wird mit 0,1 ml Plasma gestartet.
b) DiaCelin zur Messung der aktivierten partiellen Thromoplastinzeit (APTT)
   0,2 ml des Reagenz - hergestellt wie in Beispiel 1 - werden in die zweite Küvette pipettiert. Die Reaktion wird mit 0,1 ml Plasma gestartet.
c) Fibrinogenreagenz zur Bestimmung der Fibrinogenkonzentration
   Das Thrombinreagenz wird 1 + 1 mit Owren Puffer gemischt, 0,2 ml davon werden in Küvette 3 pipettiert. Die Reaktion wird mit 0,02 ml Plasma gestartet.
d) DiaThrombin zur Bestimmung der Thrombinzeit (TT)
   Das Lyophilisat wird mit 31 ml destilliertem Wasser gelöst. Davon werden 0,2 ml in die Küvette 4 pipettiert. Die Reaktion wird mit 0,2 ml Plasma gestartet.
e) DiaReptin zur Bestimmung der Reptilasezeit (RT)
   0,1 ml des Reagenz werden mit 3 ml destilliertem Wasser verdünnt. 0,2 ml der Verdünnung werden in Küvette 5 pipettiert. Gestartet wird die Reaktion mit 0,2 ml Plasma.

Die Ergebnisse von Beispiel 3 sind in Tabelle 3 zusammengefaßt und zeigen die gute Vergleichbarkeit der Methoden. Figur 5 zeigt eine Karte, mit der diese Bestimmungen gleichzeitig mit einem Patientenplasma gemacht werden können.

**Tabelle 3**

| | Gerinnungszeit (sec) | | | | | | | | | |
|---|---|---|---|---|---|---|---|---|---|---|
| | PT | | APTT | | Fib | | TT | | RT | |
| | alt | neu | alt | neu | alt | neu | alt | neu | alt | neu |
| Normal Plasma 1 | 12,0 | 11,7 | 28,3 | 89,0 | 11,5 | 11,5 | 18,5 | 19,5 | 20,8 | 20,1 |
| Abnormal Plasma 1 | 18,9 | 18,4 | 61,0 | 148,0 | 21,2 | 20,3 | 15,2 | 15,5 | 26,9 | 27,1 |
| Abnormal Plasma 2 | 27,5 | 26,5 | 97,0 | 188,0 | 22,0 | 20,7 | 15,9 | 15,9 | 33,3 | 32,7 |
| Heparin Plasma 1 | 13,0 | 13,4 | 43,0 | 110,0 | 13,4 | 13,1 | >250 | >250 | 21,3 | 20,7 |
| Heparin Plasma 2 | 13,9 | 13,7 | 82,0 | 137,0 | 13,5 | 12,6 | >250 | >250 | 19,8 | 21,3 |

### BEISPIEL 4

Das Experiment nach Beispiel 3 wird wiederholt. Die Reagenzien werden jedoch vorher in Küvetten aus Polypropylen abgefüllt und dicht mit einer Aluminiumfolie versiegelt. In Tabelle 4 sind die Ergebnisse zusammengefaßt. Es zeigt, daß eine Vorabfüllung und Verschließung der Reagenzien keine Veränderung der Resultate bewirkt.

**Tabelle 4**

| | Gerinnungszeit (Sec) | | | | | | | | | |
|---|---|---|---|---|---|---|---|---|---|---|
| | PT | | APTT | | Fib | | TT | | RT | |
| | versiegelt | normal | versiegelt | normal | versiegelt | normal | versiegelt | normal | versiegelt | normal |
| Nonual Plasma 1 | 11,8 | 11,8 | 86,8 | 88,4 | 10,8 | 10,8 | 21,0 | 21,0 | 19,8 | 19,9 |
| Abnormal Plasma 1 | 18,5 | 17,9 | 145,0 | 147,0 | 19,3 | 19,0 | 17,3 | 17,1 | 26,4 | 26,3 |
| Abnormal Plasma 2 | 26,2 | 26,7 | 185,0 | 185,0 | 17,8 | 18,1 | 17,1 | 17,4 | 30,6 | 32,3 |
| Heparin Plasma 1 | 13,5 | 13,1 | 108,0 | 109,0 | 11,4 | 11,8 | >250 | >250 | 19,9 | 21,1 |
| Heparin Plasma 2 | 13,3 | 13,2 | 137,0 | 138,0 | 12,9 | 12,2 | >250 | >250 | 20,7 | 20,0 |

## Patentansprüche

1. Verfahren zur Bestimmung der aktivierten partiellen Thromboplastinzeit (APTT) in einer Einstufen-Reaktion,
**dadurch gekennzeichnet,**
daß man die Bestimmung von APTT durch Kontaktieren eines Reagenz, das die zur APTT-Bestimmung erforderlichen Substanzen umfassend einen Aktivator, zweiwertige Kationen und Phospholipide in einem vorgemischten Zustand enthält, und welches durch eine oder mehrere Aminosäuren stabilisiert ist, mit einer Probeflüssigkeit startet und daß man die APTT durch Messung der Gerinnungszeit bestimmt.

2. Verfahren nach Anspruch 1,
**dadurch gekennzeichnet,**
daß man Plasma als Probeflüssigkeit verwendet.

3. Verfahren nach Anspruch 1 oder 2,
**dadurch gekennzeichnet,**
daß als Aktivator Kaolin, Silicat oder/und Elagsäure verwendet werden.

4. Verfahren nach Anspruch 1 oder 2,
**dadurch gekennzeichnet,**
daß die Phospholipide aus tierischen oder/und pflanzlichen Gewebeextrakten gewonnen oder/und synthetisch hergestellt sind.

5. Verfahren nach Anspruch 1 oder 2,
**dadurch gekennzeichnet,**
daß als zweiwertiges Kation Calciumionen verwendet werden.

6. Verfahren nach Anspruch 1,
**dadurch gekennzeichnet,**
daß die Aminosäuren aus der Gruppe, die D-Alanin, L-Alanin, β-Alanin, Glycin und Valin umfaßt, ausgewählt sind.

7. Verfahren nach Anspruch 6,
**dadurch gekennzeichnet,**
daß die Aminosäuren in einer Gesamtkonzentration von 0,5 bis 10% bezüglich des Reagenzgesamtgewichts vorliegen.

8. Verfahren nach einem der Ansprüche 1 bis 4,
**dadurch gekennzeichnet,**
daß das Reagenz in einem Gefäß abgepackt bzw. vorgefüllt ist.

9. Verfahren nach Anspruch 1,
**dadurch gekennzeichnet,**
daß das Gefäß luft- und wasserdicht verschlossen ist.

10. Verfahren nach Anspruch 8 oder 9,
**dadurch gekennzeichnet,**
daß der Verschluß des Gefäßes aus einer Versiegelung mit Aluminiumfolie besteht.

11. Verfahren nach einem der Ansprüche 8 bis 10,
**dadurch gekennzeichnet,**
daß mehrere Meßgefäße in einer Karte integriert sind.

12. Verfahren nach einem der Ansprüche 1 bis 11,
**dadurch gekennzeichnet,**
daß reagenzien- oder/und chargenspezifische Daten auf den Gefäßen oder/und deren Halterung gespeichert werden.

13. Verfahren nach einem der Ansprüche 9 bis 12,
**dadurch gekennzeichnet,**
daß die Speicherung auf einem Strichcode-Etikett erfolgt.

14. Verfahren nach einem der Ansprüche 1 bis 13,
**dadurch gekennzeichnet,**
daß die Bestimmungen in einem mechanischen oder optischen Analyse-system erfolgen.

15. Verfahren nach einem der Ansprüche 1 bis 14,
**dadurch gekennzeichnet,**
daß das Verfahren parallel mit weiteren Bestimmungen durchgeführt wird.

16. Verfahren nach Anspruch 15,
**dadurch gekennzeichnet,**
daß die weiteren Bestimmungen einen oder mehrere Parameter des Gerinnungssystems umfassen, die ausgewählt sind aus der Thromboplastinzeit (PT), der Fibrinogenkonzentration, der Thrombinzeit (TT), der Reptilasezeit (RT), Gerinnungsfaktoren, Protein C und Protein S.

17. Reagenz zur Bestimmung der aktivierten partiellen Thromboplastinzeit (APTT) in einer Einstufen-Reaktion,
**dadurch gekennzeichnet,**
daß es alle zur Bestimmung der APTT erforderlichen Substanzen umfassend einen Aktivator, Phospholipide und zweiwertige Kationen in vorgemischtem Zusatz enthält, und durch eine oder mehrere Aminosäuren stabilisiert ist.

18. Reagenz nach Anspruch 17,
**dadurch gekennzeichnet,**
daß es die Aminosäuren aus der Gruppe, die D-Alanin, L-Alanin, β-Alanin, Glycin und Valin umfaßt, ausgewählt sind.

19. Reagenz nach einem der Ansprüche 17 oder 18,
**dadurch gekennzeichnet,**
daß die Aminosäuren in einer Gesamtkonzentration von 0,5 bis 10% bezüglich des Reagenzgesamtgewichts vorliegen.

20. Reagenz nach einem der Ansprüche 17 bis 19,
**dadurch gekennzeichnet,**
daß es in einem Gefäß abgepackt bzw. vorgefüllt ist.

21. Reagenz nach Anspruch 20,
**dadurch gekennzeichnet,**
daß es mehrere in einer Karte integrierte Gefäße umfaßt.

22. Reagenz nach einem der Ansprüche 17 bis 21,
**dadurch gekennzeichet,**
daß reagenzien- oder/und chargenspezifische Daten auf den Gefäßen oder/und deren Halterung gespeichert sind.

23. Reagenzienkombination,
**dadurch gekennzeichnet,**
daß sie mehrere Gefäße mit einem Reagenz zur Bestimmung von APTT nach einem der Ansprüche 17 bis 22 und gegebenenfalls weitere Gefäße zur Bestimmung anderer Parameter des Gerinnungssystems umfaßt.

24. Verwendung eines Reagenz nach einem der Ansprüche 17 bis 22 oder einer Reagenzienkombination nach Anspruch 23 in einem Verfahren, bei dem die aktivierte partielle Thromboplastinzeit (APTT) in einer Probenflüssigkeit, insbesondere Blut oder Plasma, in einer Einstufen-Reaktion bestimmt wird.

## Claims

1. Method for the determination of the activated partial thromboplastin time (APTT) in a one-step reaction,
**wherein**
the determination of APTT is started by contacting a reagent which contains the substances necessary for the APTT determination comprising an activator, divalent cations and phospholipids in a premixed form and which is stabilized by one or several amino acids, with a sample liquid and determining the APTT by measuring the clotting time.

2. Method as claimed in claim 1,
**wherein**
plasma is used as the sample liquid.

3. Method as claimed in claim 1 or 2,
**wherein**
kaolin, silicate or/and ellagic acid are used as the activator.

4. Method as claimed in claim 1 or 2,
**wherein**
the phospholipids are obtained from animal or/and vegetable tissue extracts or/and are produced synthetically.

5. Method as claimed in claim 1 or 2,
**wherein**
calcium ions are used as the divalent cation.

6. Method as claimed in claim 1,
**wherein**
the amino acids are selected from the group comprising D-alanine, L-alanine, β-alanine, glycine and valine.

7. Method as claimed in claim 6,
**wherein**
the amino acids are present at a total concentration of 0.5 to 10 % in relation to the total weight of the reagent.

8. Method as claimed in one of the claims 1 to 4,
**wherein**
the reagent is packaged or pre-dispensed in a vessel.

9. Method as claimed in claim 1,
**wherein**
the vessel is sealed air- and water-tight.

10. Method as claimed in claim 8 or 9,
**wherein**
the closure of the vessel is composed of a seal made of aluminium foil.

11. Method as claimed in one of the claims 8 to 10,
**wherein**
several measuring vessels are integrated into one card.

12. Method as claimed in one of the claims 1 to 11,
**wherein**
reagent-specific or/and batch-specific data are stored on the vessels or/and on their holder.

13. Method as claimed in one of the claims 9 to 12,
**wherein**
the storage is on a bar code label.

14. Method as claimed in one of the claims 1 to 13,
**wherein**
the determinations are carried out in a mechanical or optical analytical system.

15. Method as claimed in one of the claims 1 to 14,
**wherein**
the method is carried out in parallel with further determinations.

16. Method as claimed in claim 15,
**wherein**
the further determinations comprise one or several parameters of the coagulation system that are selected from the thromboplastin time (PT), fibrinogen concentration, thrombin time (TT), reptilase time (RT), coagulation factors, protein C and protein S.

17. Reagent for the determination of the activated partial thromboplastin time (APTT) in a one-step reaction,
**wherein**
it contains all substances necessary for the determination of APTT comprising an activator, phospholipids and divalent cations in a premixed form and is stabilized by one or several amino acids.

18. Reagent as claimed in claim 17,
**wherein**
the amino acids are selected from the group comprising D-alanine, L-alanine, β-alanine, glycine and valine.

19. Reagent as claimed in one of the claims 17 or 18,
**wherein**
the amino acids are present at a total concentration of 0.5 to 10 % in relation to the total weight of the reagent.

20. Reagent as claimed in one of the claims 17 to 19,
**wherein**
it is packaged or pre-dispensed in a vessel.

21. Reagent as claimed in claim 20,
**wherein**
it comprises several vessels integrated into a card.

22. Reagent as claimed in one of the claims 17 to 21,
**wherein**
reagent-specific or/and batch-specific data are stored on the vessels or/and their holder.

23. Combination of reagents,
**wherein**
it comprises several vessels containing a reagent for determining APTT as claimed in one of the claims 17 to 22 and optionally further vessels for determining other parameters of the coagulation system.

24. Use of a reagent as claimed in one of the claims 17 to 22 or of a combination of reagents as claimed in claim 23 in a method in which the activated partial thromboplastin time (APTT) is determined in a one-step reaction in a sample liquid, in particular blood or plasma.

## Revendications

1. Procédé pour la détermination du temps de thromboplastine partielle activée (TTPA) dans une réaction à étape unique,
caractérisé en ce que l'on commence la détermination de la TTPA par mise en contact d'un réactif, comprenant les substances nécessaires pour la détermination de la TTPA contient un activateur, des cations bivalents et des phospholipides dans un état prémélangé et qui est stabilisé par un ou plusieurs acides aminés, avec un liquide d'échantillon et en ce que l'on détermine la TTPA par mesure du temps de coagulation.

2. Procédé selon la revendication 1, caractérisé en ce que l'on utilise le plasma comme liquide d'échantillon.

3. Procédé selon la revendication 1 ou 2, caractérisé en ce qu'en tant qu'activateur on utilise du kaolin, du silicate, et/ou de l'acide élagique.

4. Procédé selon la revendication 1 ou 2, caractérisé en ce que l'on prépare le phospholipide à partir d'extraits de tissus animaux et/ou végétaux et/ou de façon synthétique.

5. Procédé selon la revendication 1 ou 2, caractérisé en ce qu'en tant que cation bivalent, on utilise des ions calcium.

6. Procédé selon la revendication 1, caractérisé en ce que l'on choisit les acides aminés à partir du groupe comprenant la D-alanine, la L-alanine, la β-alanine, la glycine et la valine.

7. Procédé selon la revendication 6, caractérisé en ce que les acides aminés sont présents dans une concentration globale de 0,5 à 10 % rapportés au poids total du réactif.

8. Procédé selon l'une des revendication 1 à 4, caractérisé en ce que le réactif est conditionné ou prérempli dans un récipient.

9. Procédé selon la revendication 1, caractérisé en ce que le récipient est fermé de façon étanche à l'air et à l'eau.

10. Procédé selon la revendication 8 ou 9, caractérisé en ce que la fermeture du récipient comporte un seau avec une feuille d'aluminium.

11. Procédé selon l'une des revendications 8 à 10, caractérisé en ce que plusieurs récipients de mesures sont intégrés dans une carte.

12. Procédé selon l'une des revendications 1 à 11, caractérisé en ce que les données relatives à l'agent réactif et/ou à la charge sont mémorisées sur les récipients et/ou sur son support.

13. Procédé selon l'une des revendications 9 à 12, caractérisé en ce que la mémorisation s'effectue par le biais d'une étiquette à code barres .

14. Procédé selon l'une des revendications 1 à 13, caractérisé en ce que les déterminations s'effectuent dans un système d'analyse mécanique ou optique.

15. Procédé selon l'une des revendications 1 à 14, caractérisé en ce que le procédé est conduit parallèlement à d'autres déterminations.

16. Procédé selon la revendication 15, caractérisé en ce que les autres déterminations englobent un ou plusieurs paramètres du système de coagulation qui sont choisis à partir du temps de thromboplastine (TP), la concentration fibrinogène, du temps de thrombine (TT), du temps de reptilase (TR), des facteurs de coagulation, de la protéine C et protéine S.

17. Réactif pour la détermination du temps de thromboplastine partiel activé (TTPA) dans une réaction à étape unique, caractérisé en ce qu'il contient toutes les substances nécessaires pour la détermination de la TTPA comprenant un activateur, des phospholipides et des cations bivalents à l'état prémélangé et stabilisé par un ou plusieurs acides aminés.

18. Réactif selon la revendication 17, caractérisé en ce qu'il comprend les acides aminés choisis dans le groupe comportant la D-alanine, la L-alanine, la β-alanine, la glycine et la valine.

19. Réactif selon l'une des revendications 17 ou 18, caractérisé en ce que les acides aminés sont introduits dans une concentration globale de 0,5 à 10 % rapporté au poids total du réactif.

20. Réactif selon l'une des revendications 17 à 19, caractérisé en ce qu'il est conditionné ou prérempli dans un récipient.

21. Réactif selon la revendication 20, caractérisé en ce qu'il comprend plusieurs récipients intégrés dans une carte.

22. Réactif selon l'une des revendications 17 à 21, caractérisé en ce que les données spécifiques des réactifs et/ou de la charge sont mémorisés sur les récipients et/ou sur leur support.

23. Combinaison de réactif, caractérisé en ce qu'elle comprend plusieurs récipients avec un réactif pour la détermination de la TTPA, selon l'une des revendications 17 à 22 et éventuellement plusieurs récipients pour la détermination d'autres paramètres du système de coagulation.

24. Utilisation d'un réactif selon l'une des revendications 17 à 22, ou d'une combinaison de réactifs, selon la revendication 23, dans un procédé dans lequel on détermine le temps de thromboplastine partiel, activé TTPA dans un liquide d'échantillon, en particulier de sang ou de plasma, dans une réaction en une seule étape.
